# EUROPEAN PATENT APPLICATION

(11) **EP 4 062 955 A1**
(43) Date of publication of application: **28.09.2022**
(21) Application number: 20889409.7
(22) Date of filing: 19.11.2020
(51) Int. Cl.: A61M 3/02, A61M 31/00

(54) **AUTOMATIC CLEANING AND WASHING DEVICE FOR ADMINISTERING SUBSTANCES TO BODY**

(30) Priority: 22.11.2019 KR 20190150938
(71) Applicant: CH Bio Co., Ltd, Asan-si, Chungcheongnam-do 31499 (KR)
(72) Inventor: JEONG, Hwa, Asan-si Chungcheongnam-do 31499 (KR)
(74) Representative: AWA Sweden AB
(86) International application number: PCT/KR2020/016411
(87) International publication number: WO 2021/101286

(57) **Abstract**

An automatic cleaning and washing device for administering substances to a body is provided. An automatic cleaning and washing device for administering substances in the body, according to one embodiment, comprises: a main body that moves internal substances stored in a predetermined accommodation space to a front end electrically depending on an operation of a driving part moving forward or backward; and a nozzle part that is coupled to or separated from a connection part configured at the front end of the main body, is inserted at least partially into the human body while coupled to the connection part of the main body, and administers the internal substances into the human body through an injection hole configured at the front end depending on an operation of the driving part of the main body.

## Description

### [Technical Field]

The present invention relates to an automatic cleaning and washing device, and more particularly, to an electrically-driven automatic cleaning and washing device for administrating a substance into the human body.

### [Background Art]

In general, for the purpose of treatment and hygiene, a therapeutic agent, a washing solution, or the like in the form of a solution may be administered into a female's vagina or anus. For example, a washing solution for washing a female's vagina, a contraceptive solution, or a therapeutic drug in the form of a solution for treating hemorrhoids and sexually transmitted diseases, or a lubricant for smooth sexual intercourse may be administered to a female's vagina or anus. Since a female's vagina or anus is easily infected with bacteria through exposed mucous membranes, particular attention should be paid to cleanliness when injecting a liquid into the human body.

Due to hygiene problems and difficulty in washing, devices that administer liquids into the human body are often made for one-time use. Accordingly, such devices are sold on the market with liquids contained therein. However, when a user arbitrarily washes and reuses a disposable liquid dispenser, the user is susceptible to diseases and bacteria. Accordingly, a technique for cleaning such a liquid dispenser is required.

Korean Patent Application Publication No. 10-2003-0028796 relates to a female vagina washer and describes a technology capable of cleaning and sterilizing the vagina using a syringe equipped with a long nozzle.

### [Disclosure]

### [Technical Problem]

Therefore, the present invention has been made in view of the above problems, and it is one object of the present invention to provide an automatic cleaning and washing device for administrating a substance into the human body, and more particularly, to provide a technology related to an electrically-driven automatic cleaning and washing device for administrating a substance, such as a vagina cleansing solution, a liquid medicine, or a lubricant, into a female's vagina or anus.

It is another object of the present invention to provide an automatic cleaning and washing device for administrating a substance into the human body, wherein the device includes a nozzle part and a main body that are configured so that the nozzle part is selectively coupled to the main body depending on a gel, liquid, or tablet (capsule) type, and the device is electrically driven to minimize shaking when pressed to administer a substance into the human body.

### [Technical Solution]

In accordance with one aspect of the present invention, provided is an automatic cleaning and washing device for administrating a substance into a human body, including a main body for electrically moving an internal substance stored in a predetermined accommodation space to a front end depending on operation of a driving unit moving forward or backward; and a nozzle part configured to be coupled to or separated from a coupling part provided at a front end of the main body, wherein at least a portion of the nozzle part is inserted into a human body in a state in which the nozzle part is coupled to the coupling part of the main body, and the nozzle part injects the internal substance into the human body through an injection hole provided at a front end thereof depending on operation of a driving unit of the main body.

The main body may include a control unit for controlling power and operation of the driving unit through button manipulation by a user; and a power supply unit for supplying power to operate the driving unit, wherein the control unit includes a power control unit for controlling operation of the power supply unit; an operation mode selection unit for selecting motors of the driving unit to control operation modes; and a strength selection unit for adjusting a rotation speed of the selected motors to control strength.

The main body may further include a display unit for displaying remaining battery amount, operation modes, and strength according to states of the control unit and the power supply unit.

The main body is capable of operating in a washing mode or a cleaning mode by selectively operating at least one of two motors moving forward or backward, wherein, when the washing mode using a washing agent is selected, washing motors operate to move a washing pinion forward to discharge the internal substance for a predetermined time, and after discharging is completed, the washing motors rotate in a reverse direction to return to an original position thereof; and when a cleaning mode using a cleaning agent is selected, the washing motors and a cleaning motor operate simultaneously to move the washing pinion and a cleaning pinion forward, the washing motors are stopped after moving by a specified number of revolutions or a specified distance, the cleaning motor continues to move forward to move a cleaning push rod to a front end point to discharge the internal substance for a predetermined time, and after discharging is completed, the washing motors and the cleaning motor rotate in a reverse direction to return to original positions thereof.

The automatic cleaning and washing device may further include a cap coupled to an outside of the nozzle part to protect the nozzle part from external factors, wherein the coupling part of the main body is composed of a wrinkle part made of a flexible material, so that the coupling part is freely bent during use to change a direction of the nozzle part; and the nozzle part is selectively coupled to the main body according to types (gel, liquid, or tablet (capsule)) of the washing agent and the cleaning agent.

### [Advantageous effects]

According to embodiments, the present invention can provide an electrically-driven automatic cleaning and washing device for administrating a substance into the human body, wherein the device is used to administer a vagina cleansing solution, a liquid medicine, or a lubricant into a female's vagina or anus and is capable of minimizing shaking when pressed to administer a substance into the human body.

According to embodiments, the present invention can provide an automatic cleaning and washing device for administrating a substance into the human body, wherein the device includes a nozzle part and a main body that are configured so that the nozzle part is selectively coupled to the main body depending on a gel, liquid, or tablet (capsule) type, and to improve hygiene, the device is configured so that the nozzle and the substance do not come into contact with the human body, including the hand, when the substance is administered into the human body.

### [Description of Drawings]

FIG. 1 is a front view of an automatic cleaning and washing device including a case according to one embodiment.
FIG. 2 is a front view of an automatic cleaning and washing device including a first nozzle part according to one embodiment.
FIG. 3 is a front view of an automatic cleaning and washing device including a second nozzle part according to one embodiment.
FIG. 4 is a front view of an automatic cleaning and washing device including a third nozzle part according to one embodiment.
FIG. 5 illustrates the coupling part of an automatic cleaning and washing device according to one embodiment.
FIG. 6 is a drawing for explaining disassembly and assembly of an automatic cleaning and washing device according to one embodiment.
FIG. 7 is a front view of an automatic cleaning and washing device including a nozzle cover part according to one embodiment.
FIG. 8 is a front view of an automatic cleaning and washing device including a battery part according to one embodiment.
FIG. 9 is a side view of the main body of an automatic cleaning and washing device according to one embodiment.
FIG. 10 is a drawing for explaining coupling and disassembly of a pump part inserted into the main body of an automatic cleaning and washing device according to one embodiment.
FIG. 11 is a side cross-sectional view of an automatic cleaning and washing device according to one embodiment showing a position of the automatic cleaning and washing device before use for cleaning.
FIG. 12 is a side cross-sectional view of an automatic cleaning and washing device according to one embodiment showing a position of the automatic cleaning and washing device after use for cleaning.
FIG. 13 is a side cross-sectional view of an automatic cleaning and washing device according to one embodiment showing a cleaning use position of the automatic cleaning and washing device.
FIG. 14 is a side cross-sectional view of an automatic cleaning and washing device according to one embodiment showing a moved position of the automatic cleaning and washing device after use for cleaning.
FIG. 15 is a front cross-sectional view showing motors and gears for washing and cleaning according to one embodiment.
FIG. 16 is a drawing for explaining operation buttons according to one embodiment.

### [Best mode]

Hereinafter, preferred embodiments of the present invention will be described in detail with reference to the accompanying drawings. However, the invention may be embodied in many different forms and should not be construed as being limited to the embodiments set forth herein. These embodiments are provided to more fully describe the present invention to those skilled in the art. The shapes and sizes of elements in the drawings may be exaggerated for clearer description.

The following embodiments relate to an automatic cleaning and washing device for administrating a substance into the human body. By providing an electrically-driven automatic cleaning and washing device for administering a substance, such as a vagina cleansing solution, a liquid medicine, or a lubricant, into a female's vagina or anus, shaking may be minimized when pressed to administer the substance into the human body. In addition, depending on a gel, liquid, or tablet (capsule) type, a nozzle part may be selectively coupled to a main body, and the nozzle part and the substance do not come into contact with the hand when the substance is administered into the human body, thereby improving hygiene.

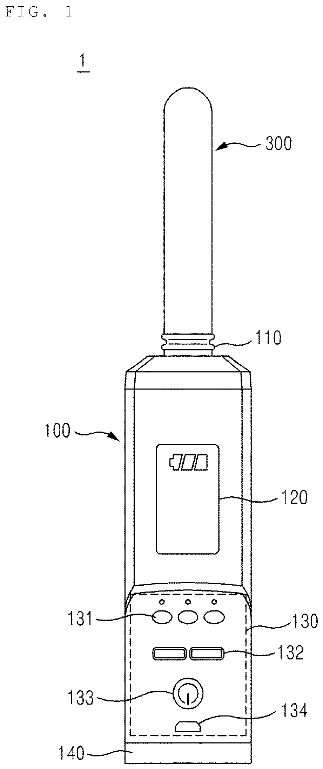

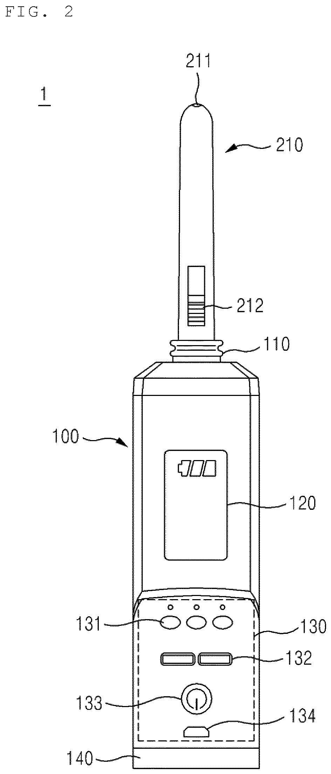

FIG. 1 is a front view of an automatic cleaning and washing device including a case according to one embodiment, and FIG. 2 is a front view of an automatic cleaning and washing device including a first nozzle part according to one embodiment.

Referring to FIGS. 1 and 2, an automatic cleaning and washing device 1 for administrating a substance into the human body according to one embodiment may include a main body 100 and a nozzle part 210. According to an embodiment, the automatic cleaning and washing device 1 may further include a cap 300 coupled to the outside of the nozzle part 210 to protect the nozzle part 210 from external environments.

A predetermined accommodation space for accommodating a material such as a liquid may be formed inside the main body 100, and a driving unit for discharging or ejecting an internal substance to the outside may be provided inside the main body 100. That is, the main body 100 may electrically move an internal substance stored in the predetermined accommodation space to the front end depending on operation of the driving unit moving forward or backward. For example, the driving unit may include two motors that are electrically driven forward and backward, which are used in electric toothbrushes and bidets.

In addition, the front end of the main body 100 may be provided with a coupling part 110 for coupling to or separation from the nozzle part 210. In this case, the coupling part 110 of the main body 100 may be composed of a wrinkle part made of a flexible material. Accordingly, the coupling part 110 is freely bent during use, so that the direction of the nozzle part 210 may be changed.

The main body 100 may include a control unit 130 for controlling power and operation of a driving unit through button manipulation by a user and a power supply unit 140 for supplying power to operate the driving unit.

The control unit 130 may include the power control unit 130 for controlling operation of the power supply unit 140, an operation mode selection unit for selecting the motor of a driving unit to control an operation mode, and a strength selection unit for controlling strength (weak, medium, strong) by adjusting the rotation speed of the selected motor. In addition, the control unit 130 may further include a charging terminal for power charging .

For example, the power control unit 130 may control power on/off, the operation mode selection unit may select a cleaning mode or a washing mode, and the strength selection unit may select discharge strength (strong, medium, weak) of an internal substance.

In addition, the main body 100 may further include a display unit for displaying a remaining battery amount, an operation mode, and strength according to the states of the control unit 130 and the power supply unit 140. The display unit may be configured as an LCD window.

The main body 100 is capable of operating in a washing mode or a cleaning mode by selectively operating at least one of two motors moving forward or backward. For example, when selecting a washing mode using a vaginal cleanser, washing motors 105 operate to move a washing pinion 101 forward to discharge an internal substance for a predetermined time. After discharge, the washing motors 105 rotate in the reverse direction to return to the original position thereof. When selecting a cleaning mode using a feminine cleanser, the washing motors 105 and a cleaning motor 106 operate at the same time to move the washing pinion 101 and a cleaning pinion 102 forward, the washing motors 105 are stopped after moving by a specified number of revolutions or distance, and the cleaning motor 106 continues to move forward to move a cleaning push rod to the front end point so that an internal substance is discharged for a predetermined time. After discharge, the washing motors 105 and the cleaning motor 106 rotate in the reverse direction to return to the original positions thereof.

The nozzle part 210 is configured to be coupled to or separated from the coupling part 110 provided at the front end of the main body 100 and is formed in a cylindrical or streamlined shape so that a portion of the nozzle part 210 is inserted into the human body while being coupled to the coupling part 110 of the main body 100. In addition, in a state in which the nozzle part 210 is inserted into the human body, the nozzle part 210 may inject an internal substance into the human body through an injection hole 211 provided at the front end depending on operation of the driving unit of the main body 100.

The nozzle part 210 may be selectively coupled to the main body 100 according to the type (gel, liquid, or tablet (capsule)) of a feminine cleanser or a vaginal cleanser.

For example, as shown in FIG. 2, a first nozzle part 210 may be formed in a streamlined shape and may be coupled to the front end of the main body 100, and the injection hole 211 may be provided at the front end of the first nozzle part 210 so that a gel-type or liquid-type internal substance may be discharged to the outside. At this time, as the gel-type or liquid-type internal substance, a cleaner or multipurpose substance may be used. In the case of a liquid-type substance, since a separate tube containing the liquid is required for the nozzle part 210, a separate tube containing the liquid may be coupled to the main body 100 through an opening at the top of the main body 100. At this time, the tube may be a pleated pump configured to discharge an internal substance to the outside by compression. The nozzle part 210 described herein may include the first nozzle part 210, a second nozzle part 220, and a third nozzle part 230. For convenience, the first nozzle part 210 is mainly described.

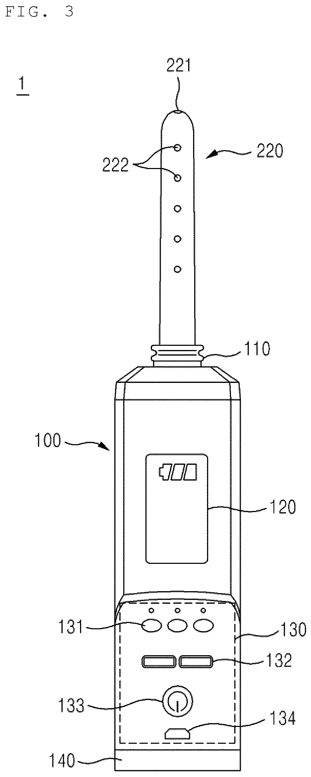

FIG. 3 is a front view of an automatic cleaning and washing device including a second nozzle part according to one embodiment.

As shown in FIG. 3, the second nozzle part 220 may be formed in a streamlined shape and may be coupled to the front end of the main body 100. The injection hole 221 may be provided at the front end of the second nozzle part 220, and a plurality of auxiliary injection holes 222 may be formed in the outer peripheral surface of the nozzle part 210 to discharge a gel-type or liquid-type internal substance to the outside. As the gel-type or liquid-type internal substance, a cleaning agent may be used.

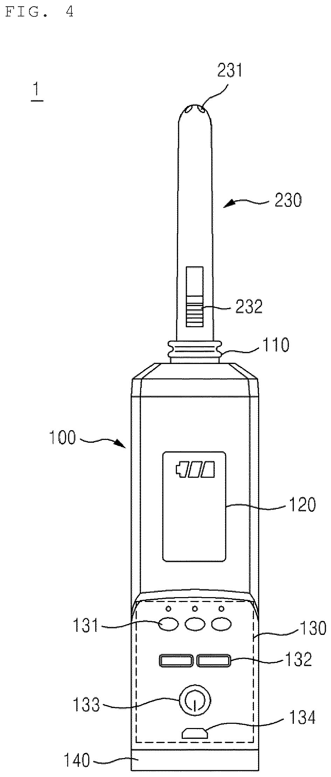

FIG. 4 is a front view of an automatic cleaning and washing device including a third nozzle part according to one embodiment.

As shown in FIG. 4, the third nozzle part 230 may be formed in a streamlined shape and may be coupled to the front end of the main body 100. Injection holes 231 composed of a plurality of slots in the shape of a cross may be provided at the front end of the third nozzle part 230 to discharge a tablet (capsule)-type internal substance to the outside. The third nozzle part 230 has a shape in which the injection hole 211 of the nozzle part 210 opens and a content come out when a push bar is pressed while a table or a capsule is contained therein. At this time, as the tablet (capsule)-type internal substance, a vaginal tablet may be used.

As described above, the tablet-type third nozzle part 230 may be coupled to an electric device and may be used in a button press manner. Accordingly, even a first-time user may operate the tablet-type third nozzle part 230 easily.

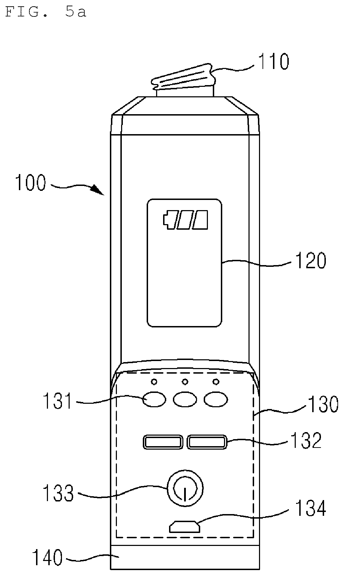

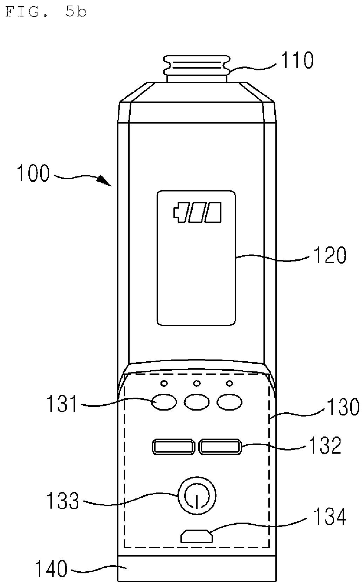

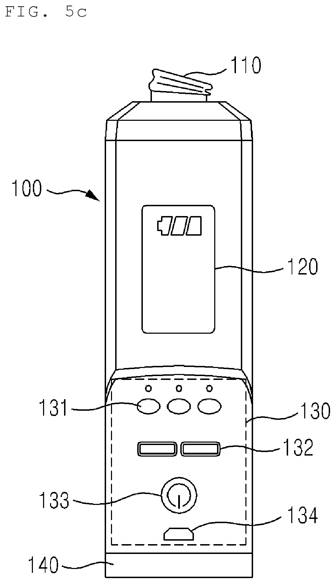

FIG. 5 illustrates the coupling part of an automatic cleaning and washing device according to one embodiment. More specifically, FIGS. 5A to 5C show that the coupling part 110 of the automatic cleaning and washing device 1 is bendable. That is, FIG. 5A shows a state in which the left side of the coupling part 110 is bent, FIG. 5B shows a state in which the coupling part 110 is not bent, and FIG. 5C shows a state in which the right side of the coupling part 110 is bent.

Referring to FIGS. 5A to 5C, the coupling part 110 of the automatic cleaning and washing device 1 may be configured to be coupled to or separated from the nozzle part 210 at the front end of the main body 100. In this case, the coupling part 110 may be composed of a wrinkle part made of a flexible material. Thus, the coupling part 110 may be freely bent during use, so that the direction of the nozzle part 210 may be changed. For example, the nozzle, the main body 100, and the coupling part 110 may be made of a flexible silicone material, and thus free bending is possible during use.

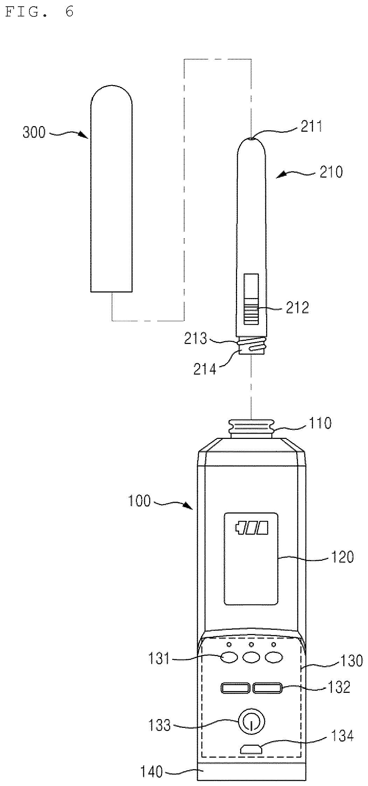

FIG. 6 is a drawing for explaining disassembly and assembly of an automatic cleaning and washing device according to one embodiment.

Referring to FIG. 6, in the automatic cleaning and washing device 1, the nozzle part 210 may be coupled to or separated from the coupling part 110 at the front end of the main body 100. The cap 300 having an open lower side may be put on the upper end of the nozzle part 210 so that the cap 300 and the nozzle part 210 are coupled.

For example, a screw part 213 for coupling with the coupling part 110 of the main body 100 may be formed at the lower end of the nozzle part 210. Accordingly, the nozzle part 210 may be coupled to or separated from the coupling part 110 of the main body 100 through screw coupling. In addition, a rubber stopper 214 may be provided at the lower end of the nozzle part 210. The rubber stopper 214 serves to prevent leakage of contents in the nozzle. In addition, the rubber stopper 214 may also serve to push contents in the nozzle when the push bar moves forward inside the nozzle. Here, the push bar may move forward depending on operation of the driving unit to discharge an internal substance to the outside through the injection hole 211 of the nozzle part 210.

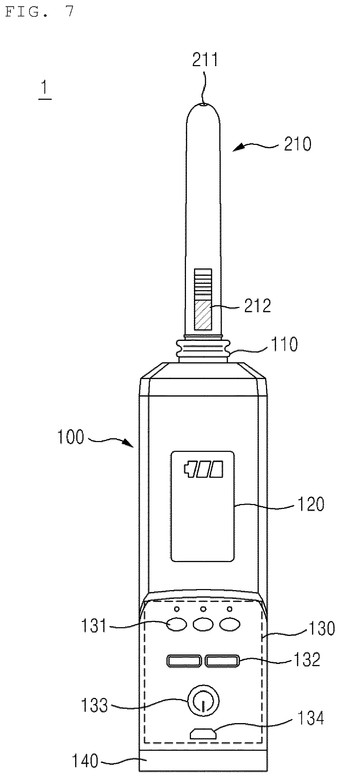

FIG. 7 is a front view of an automatic cleaning and washing device including a nozzle cover part according to one embodiment.

Referring to FIG. 7, a sliding cover 212 at the lower end of the nozzle part 210 may be opened, and an additional substance may be put through sliding cover 212 in addition to an internal substance contained in the main body 100. For example, in the case of the first nozzle part 210, in addition to the existing cleaning agents, by filling products that may be injected by moving the push bar forward, such as ointments, various products may be used. In addition, in the case of the third nozzle part 230, in addition to the existing vaginal tablets, other tablet-type products may be used.

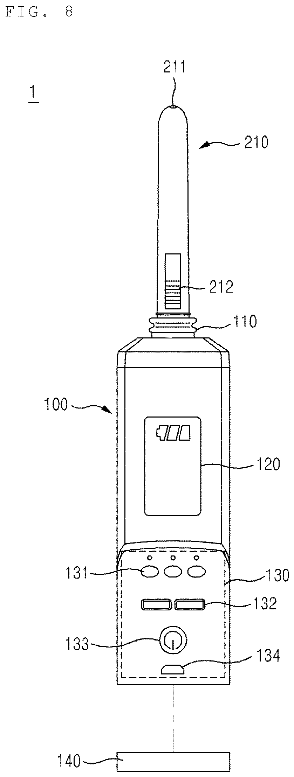

FIG. 8 is a front view of an automatic cleaning and washing device including a battery part according to one embodiment.

As shown in FIG. 8, the power supply unit 140 for operating the driving unit by supplying power may be provided at the lower end of the main body 100. The power supply unit 140 may include a rechargeable battery or a battery that does not need to be charged, or both a rechargeable battery and a battery that does not need to be charged. At this time, separate charging is not required by opening a battery socket cover and inserting a battery such as a dry cell or a mercury battery. The rechargeable battery consists of an internal battery and may be charged through a USB charging port, such as a smartphone cable.

The automatic cleaning and washing device 1 including the battery part may be implemented as a portable wireless product.

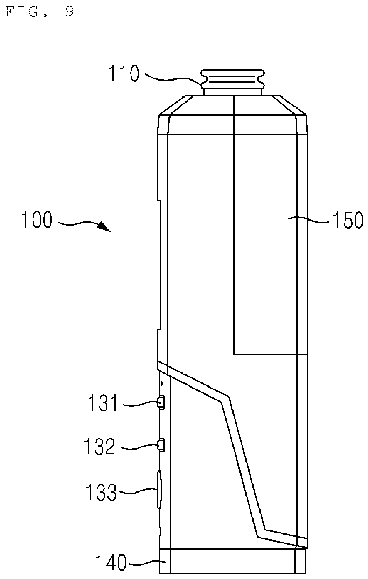

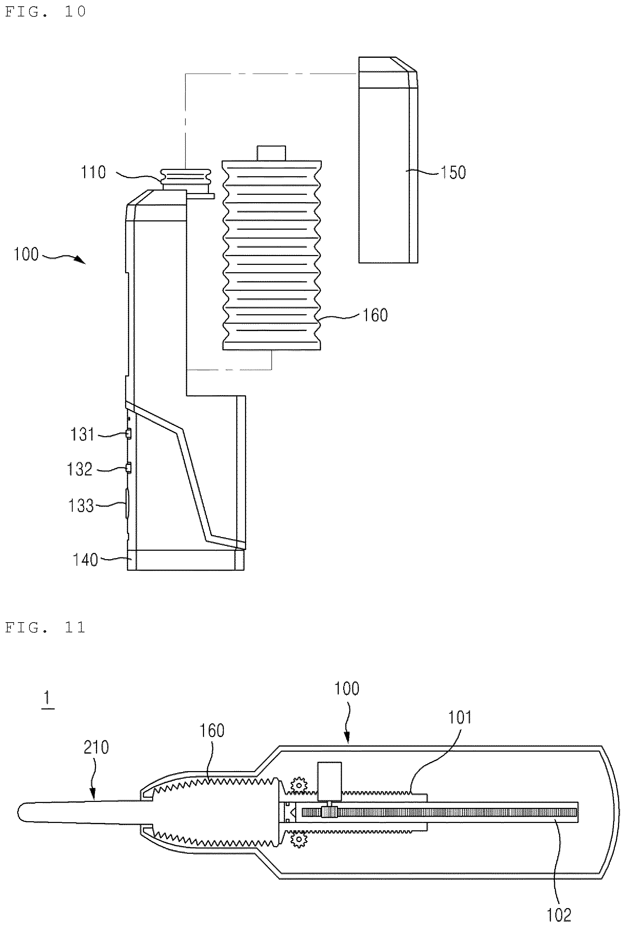

FIG. 9 is a side view of the main body of an automatic cleaning and washing device according to one embodiment, and FIG. 10 is a drawing for explaining coupling and disassembly of a tube inserted into the main body of an automatic cleaning and washing device according to one embodiment.

Referring to FIGS. 9 and 10, the main body 100 may further include a rear cover part 150, and the rear cover part 150 may be coupled or released in a vertical sliding manner to place a tube in the main body 100 before use in a cleaning mode. At this time, a tube 160 may be configured as a pleated pump to discharge an internal substance to the outside by compression. For example, the substance inside the tube 160 may be a cleaning agent.

Hereinafter, operation of a washing mode and a cleaning mode will be described with reference to FIGS. 11 to 16.

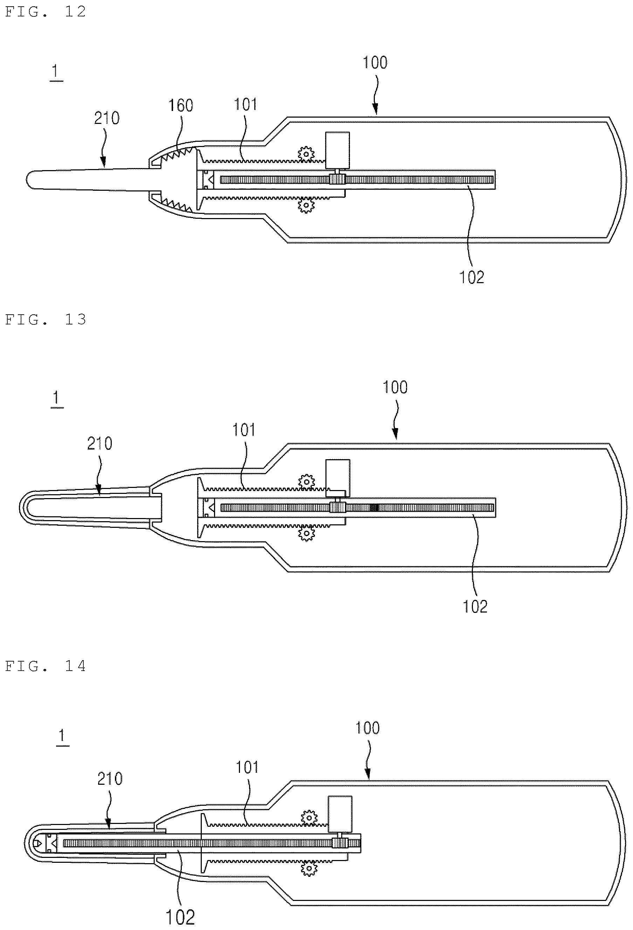

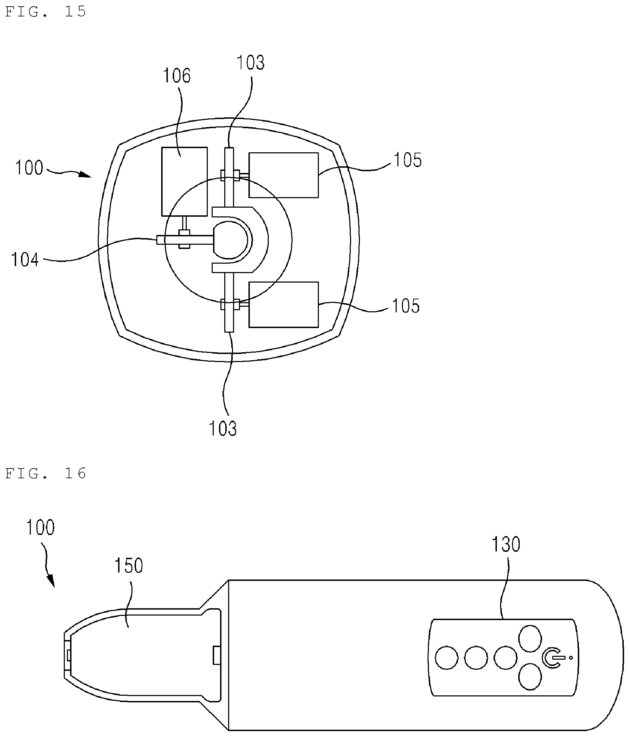

FIG. 11 is a side cross-sectional view of an automatic cleaning and washing device according to one embodiment showing a position of the automatic cleaning and washing device before use for cleaning, and FIG. 12 is a side cross-sectional view of an automatic cleaning and washing device according to one embodiment showing a position of the automatic cleaning and washing device after use for cleaning. FIG. 15 is a front cross-sectional view showing motors and gears for washing and cleaning according to one embodiment, and FIG. 16 is a drawing for explaining operation buttons according to one embodiment.

Referring to FIGS. 11, 12, 15, and 16, in the automatic cleaning and washing device 1, in a washing mode, when power is applied and a washing mode button is pressed, the washing motors 105 operate to move the washing pinion 101 forward. At this time, in the washing mode, strong, medium, or weak strength may be selected, and the strength may be controlled by the rotation speed of the washing motors 105. Contents are slowly discharged for a predetermined time (e.g., several minutes). After the discharge is complete, the motors rotate in the reverse direction to return to the original position thereof and wait for the next operation.

FIG. 13 is a side cross-sectional view of an automatic cleaning and washing device according to one embodiment showing a cleaning use position of the automatic cleaning and washing device, and FIG. 14 is a side cross-sectional view of an automatic cleaning and washing device according to one embodiment showing a moved position of the automatic cleaning and washing device after use for cleaning.

Referring to FIGS. 13 to 16, in the automatic cleaning and washing device 1, in a cleaning mode, when power is applied and a cleaning button is pressed, the washing motors 105 and the cleaning motor 106 operate at the same time to move the washing pinion 101 and the cleaning pinion 102 forward. The washing motors 105 stop after moving by a specified number of revolutions or a specified distance, and the cleaning motor 106 continues to move forward and stops after moving a cleaning push rod to the front end point. At this time, in the cleaning mode, strong, medium, or weak strength may be selected, and the strength may be controlled by the rotation speed of the motor. Contents are slowly discharged for several minutes. After the discharge is complete, the motors rotate in the reverse direction to return to the original position thereof and wait for the next operation.

The automatic cleaning and washing device 1 includes motors that only move forward and backward. In the automatic cleaning and washing device 1, various types of the nozzle parts 210 provided with the cap 300 may be coupled to an electric device corresponding to the main body 100. The nozzle part 210 consists of the cap 300, the nozzle part 210, and the rubber stopper 214. After the nozzle part 210 is inserted into the main body 100 in a screw-coupled manner, the cap 300 is removed by tapping and a desired button is pressed.

For a convenient posture during use, a wrinkled rubber is installed on the front side of the main body 100 to enable movement up, down, left, and right. An opening and closing device for filling contents is installed on the upper surface of the lower end of the nozzle part 210.

Buttons for adjusting strength (strong, medium, weak) according to user's preference are provided. An LED display is provided on the front of the electric device to display strong, medium, and weak strength and the remaining battery level, so that the device may be conveniently used even in the dark. In addition, to prevent slipping and easy gripping, a material such as rubber is provided on the outside of the device.

As described above, the feminine cleanser and the vaginal cleanser may be a gel, liquid, or tablet (capsule) type. The nozzle part 210 is provided to conveniently administer various types (gel, liquid, tablet (capsule)) of substances into the human body. The device is configured so that the nozzle part 210 and the substance do not come into contact with the hand when the substance is administered into the human body, thereby improving hygiene.

In addition, to solve the disadvantages of the existing method of applying strong force when administering a large amount of a cleaning agent, there is no need to apply strong force during pressurization, and shaking is minimized, so that injuries caused by shaking during pressurization may be prevented.

Since the main body 100 that may use all of gel, liquid, and tablet (capsule) type materials is made of a single electric device, there are advantages such as portability, ease of use, and improved hygiene. The automatic cleaning and washing device 1 may be used semi-permanently through charging and battery replacement. The automatic cleaning and washing device 1 does not include a push bar, a handle, and the like. In addition, in the case of the automatic cleaning and washing device 1, a container may be directly filled with various substances (gel, liquid, tablet) for antibacterial, anti-inflammatory, moisturizing, or elasticity purposes according to a user's request. When the substance is mass-produced while being filled, cost and consumer price may be reduced.

It should be understood that when an element is referred to as being "connected to" or "coupled to" another element, the element may be directly connected or coupled to the other element or intervening elements may be present. In contrast, when an element is referred to as being "directly connected to" or "directly coupled to" another element, there are no intervening elements present.

The terms used in the present specification are used to explain a specific exemplary embodiment and not to limit the present inventive concept. Thus, the expression of singularity in the present specification includes the expression of plurality unless clearly specified otherwise in context. Also, terms such as "include" or "comprise" should be construed as denoting that a certain characteristic, number, step, operation, constituent element, component or a combination thereof exists and not as excluding the existence of or a possibility of an addition of one or more other characteristics, numbers, steps, operations, constituent elements, components or combinations thereof.

The terms such as "first" and "second" are used herein merely to describe a variety of constituent elements, but the constituent elements are not limited by the terms. The terms are used only for the purpose of distinguishing one constituent element from another constituent element.

Terms, such as "unit" or "module", etc., should be understood as a unit that processes at least one function or operation and that may be embodied in a hardware manner, a software manner, or a combination of the hardware manner and the software manner.

In addition, components of the embodiment described with reference to each drawing are not limitedly applied only to the embodiment, and the components may be implemented to be included in other embodiments within the scope of maintaining the technical spirit of the present invention. Also, although a separate description is omitted, a plurality of embodiments may be re-implemented as a single integrated embodiment.

In addition, the present invention will be described in detail by explaining exemplary embodiments of the invention with reference to the attached drawings. In the drawings, the same elements are denoted by the same reference numerals, and a repeated explanation thereof will not be given. In addition, in the following description of the present invention, a detailed description of known functions and configurations incorporated herein will be omitted when it may make the subject matter of the present invention unclear.

Although the present invention has been described with reference to limited embodiments and drawings, it should be understood by those skilled in the art that various changes and modifications may be made therein. For example, the described techniques may be performed in a different order than the described methods, and/or components of the described systems, structures, devices, circuits, etc., may be combined in a manner that is different from the described method, or appropriate results may be achieved even if replaced by other components or equivalents.

Therefore, other embodiments, other examples, and equivalents to the claims are within the scope of the following claims.

## Claims

1. An automatic cleaning and washing device for administrating a substance into a human body, comprising:
a main body for electrically moving an internal substance stored in a predetermined accommodation space to a front end depending on operation of a driving unit moving forward or backward; and
a nozzle part configured to be coupled to or separated from a coupling part provided at a front end of the main body,
wherein at least a portion of the nozzle part is inserted into a human body in a state in which the nozzle part is coupled to the coupling part of the main body, and the nozzle part injects the internal substance into the human body through an injection hole provided at a front end thereof depending on operation of a driving unit of the main body.

2. The automatic cleaning and washing device according to claim 1, wherein the main body comprises a control unit for controlling power and operation of the driving unit through button manipulation by a user; and a power supply unit for supplying power to operate the driving unit,
wherein the control unit comprises a power control unit for controlling operation of the power supply unit; an operation mode selection unit for selecting motors of the driving unit to control operation modes; and a strength selection unit for adjusting a rotation speed of the selected motors to control strength.

3. The automatic cleaning and washing device according to claim 2, wherein the main body further comprises a display unit for displaying remaining battery amount, operation modes, and strength according to states of the control unit and the power supply unit.

4. The automatic cleaning and washing device according to claim 1, wherein the main body is capable of operating in a washing mode or a cleaning mode by selectively operating at least one of two motors moving forward or backward,
wherein, when the washing mode using a washing agent is selected, washing motors operate to move a washing pinion forward to discharge the internal substance for a predetermined time, and after discharging is completed, the washing motors rotate in a reverse direction to return to an original position thereof; and
when a cleaning mode using a cleaning agent is selected, the washing motors and a cleaning motor operate simultaneously to move the washing pinion and a cleaning pinion forward, the washing motors are stopped after moving by a specified number of revolutions or a specified distance, the cleaning motor continues to move forward to move a cleaning push rod to a front end point to discharge the internal substance for a predetermined time, and after discharging is completed, the washing motors and the cleaning motor rotate in a reverse direction to return to original positions thereof.

5. The automatic cleaning and washing device according to claim 1, further comprising a cap coupled to an outside of the nozzle part to protect the nozzle part from external factors, wherein the coupling part of the main body is composed of a wrinkle part made of a flexible material, so that the coupling part is freely bent during use to change a direction of the nozzle part; and the nozzle part is selectively coupled to the main body according to types (gel, liquid, or tablet (capsule)) of the washing agent and the cleaning agent.
